# EUROPEAN PATENT APPLICATION

(11) **EP 3 967 308 A1**
(43) Date of publication of application: **16.03.2022**
(21) Application number: 20306009.0
(22) Date of filing: 11.09.2020
(51) Int. Cl.: A61K 31/44, A61P 21/00

(54) **MEPYRAMINE FOR USE IN THE TOPICAL TREATMENT OF NEUROPATHIC PAIN**

(71) Applicant: ASSISTANCE PUBLIQUE, HOPITAUX DE PARIS, 75004 Paris (FR); Centre national de la recherche scientifique, 75016 Paris (FR)
(72) Inventor: GRECO, Céline, 75012 PARIS (FR); DELMAS, Patrick, 13880 VELAUX (FR)
(74) Representative: Gevers & Orès

(57) **Abstract**

The present invention relates to a compound selected from mepyramine, prodrugs thereof and pharmaceutically acceptable salts thereof, or a composition comprising such a compound, for use in the topical treatment and prevention of neuropathic pain.

## Description

The present invention relates to a compound selected from mepyramine, prodrugs thereof and pharmaceutically acceptable salts thereof, or a composition comprising such a compound, for use in the topical treatment and prevention of neuropathic pain.

Neuropathic pain is caused by a lesion or a disease of the somatosensory system, including peripheral fibers and central neurons, and affects 7-10% of the general population. Multiple causes of neuropathic pain have been described and its incidence is likely to increase owing to the ageing global population. Neuropathic pain does not start abruptly or resolve quickly; it is a chronic condition which leads to persistent pain symptoms. Neuropathic pain is associated with increased drug prescriptions and visits to health care providers. Patients typically suffer from distinct painful sensations such as tingling, prickling, or pain described as shooting, stabbing, burning, or having an electric shock, or pain resulting from non-painful stimulations (such as light touching). Sleep disturbances, anxiety and depression are frequent and severe in patients with neuropathic pain, and quality of life is more impaired in patients with chronic neuropathic pain than in those with chronic non-neuropathic pain that does not come from damaged or irritated nerves.

Patients suffering from neuropathic pain typically do not respond to traditional analgesics (paracetamol, nonsteroidal anti-inflammatory drugs) or weak opioids because these do not focus on treating the types of symptoms associated with neuropathic pain. Many patients do not achieve satisfactory pain relief even with evidence-based treatment, or do not tolerate effective doses because of adverse effects. When medications fail to provide relief, interventional therapies may be considered in select patients with refractory neuropathic pain.

There thus remains a genuine need for an alternative and effective treatment having no or a less side effects compared with actual treatments.

The present invention is believed to meet such need by providing a topical composition for treating or preventing neuropathic pain.

Mepyramine is a first generation antihistamine of formula (I):

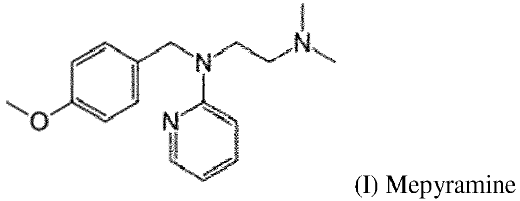

Mepyramine is classically used as a histamine H1 receptor inverse agonist. It binds to a G protein-coupled form of the receptor and promotes a G protein-coupled inactive state of the H1 receptor that interferes with the Gq/11-mediated signaling. Mepyramine competes with histamine for binding at HI-receptor sites on the effector cell surface, resulting in suppression of histaminic edema, flare, and pruritus.

The Inventors have in a surprising manner demonstrated that mepyramine has an inhibitory effect on the sodium channel Nav1.7, which is believed to have a central role in pain sensing, and that this compound can be used in a topical treatment and prevention of neuropathic pain with no side effects compared with actual treatments.

Thus, the present invention concerns a compound selected from mepyramine, prodrugs thereof and pharmaceutically acceptable salts thereof, for use in the topical treatment or prevention of neuropathic pain.

The present invention further relates to a topical composition comprising a compound according to the invention as active ingredient and at least one pharmaceutically acceptable excipient, for use in the treatment or prevention of neuropathic pain.

The present invention further relates to a method for treating and/or preventing neuropathic pain by means of topical administration, to a patient in need thereof, of an effective amount of a compound or composition according to the invention.

The present invention further relates to the use of a compound or composition comprising a compound according to the invention for the manufacture of a topical medication for treating and/or preventing neuropathic pain.

"Mepyramine" (also called "pyrilamine") as used herein refers to the molecule N,N-dimethyl-N'-(4-methoxybenzyl)-N'-(2-pyridyl) ethylenediamine, enantiomers, racemic mixtures, polymorphs, salts, solvates, esters or hydrates thereof.

In the present invention, "prodrug" designates a compound that, after administration, is metabolized (*i.e.* converted within the body) into a pharmacologically active drug.

In the present invention, "pharmaceutically acceptable" is intended to mean that which is useful in the preparation of a pharmaceutical composition, generally safe, nontoxic and neither biologically nor otherwise undesirable, and acceptable for both veterinary and human pharmaceutical use.

"Pharmaceutically acceptable salt" of a compound is intended to mean a salt that is pharmaceutically acceptable, as defined herein, and that has the desired pharmacological activity of the parent compound.

Salts of mepyramine include salts of acidic or basic groups present in compounds of the application. Pharmaceutically acceptable salts of mepyramine include, but are not limited to, mepyramine maleate (which is commercially available under the Trade Name "Anthisan") or pyrilamine acetate.

In the present invention, the term "neuropathic pain" designates a pain caused by a lesion or disease of the somatosensory nervous system.

In an embodiment, the neuropathic pain is caused by a disease chosen from the group comprising erythromelalgia, chemotherapy-induced peripheral neuropathy (CIPN), hereditary neuropathy with pressure palsies (HNPP), algoneurodystrophy, diabetic neuropathy, anti-MAG peripheral neuropathy, amyloid neuropathy, systemic mastocytosis and drug-induced peripheral neuropathy.

"Erythromelalgia" (or "erythermalgia") is a rare condition that primarily affects the feet and, less commonly, the hands. It is characterized by intense, burning pain of affected extremities, severe redness (erythema), and increased skin temperature. Recent reports have revealed an involvement of efferent small nerve fibers indicating a neuropathic component. Erythermalgia may appear to occur randomly for unknown reasons (sporadically) or may be familial, suggesting autosomal dominant inheritance. Primary erythromelalgia has been related to mutations of the voltage-gated sodium channel α-subunit gene *SCN9A* which encodes the voltage-gated sodium channel subtype Nav1.7.

"Chemotherapy-induced peripheral neuropathy" (or "CIPN") is one of the most frequent side effects caused by antineoplastic agents. Due to its high prevalence among cancer patients, CIPN constitutes a major problem for both cancer patients and survivors. There are six main substance groups that cause damage to peripheral sensory, motor and autonomic neurons, which result in the development of CIPN: platinum-based antineoplastic agents, vinca alkaloids, epothilones (ixabepilone), taxanes, proteasome inhibitors (bortezomib) and immunomodulatory drugs (thalidomide). Clinically, sensory symptoms usually develop first, involve the feet and hands and commonly present as a typical "glove and stocking" neuropathy with the most distal parts of the limbs exhibiting the greatest deficits. The symptoms comprise numbness, tingling, altered touch sensation, impaired vibration, paresthesias and dysesthesias induced by touch and warm or cool temperatures. Moreover, painful sensations, including spontaneous burning, shooting or electric shock-like pain as well as mechanical or thermal allodynia or hyperalgesia frequently occur. In severe cases, these symptoms can progress to a loss of sensory perception.

"Hereditary neuropathy with pressure palsies" (or "HNPP") is a hereditary disorder in which nerves become increasingly sensitive to pressure, injury, and use. Pressure on the nerves can cause tingling sensations, numbness, pain, weakness, muscle atrophy and even paralysis of the affected area.

"Algoneurodystrophy" (or "Regional Pain Syndrome Complex" or "SDRC") is a painful disease characterized by erythema, edema, functional impairment, sensory and vasomotor disturbance. The diagnosis of CRPS is based solely on clinical signs and symptoms, and for exclusion compared to other forms of chronic pain.

"Diabetic neuropathy" is a serious diabetes complication that may affect as many as 50% of people with diabetes. Diabetic neuropathy most often damages nerves in legs and feet. Depending on the affected nerves, diabetic neuropathy symptoms can range from pain and numbness in the legs and feet to problems with the digestive system, urinary tract, blood vessels and heart.

"Anti-MAG peripheral neuropathy" is a very rare disease that occurs when the body's own immune system develops antibodies against a key glycoprotein (myelin-associated glycoprotein, or MAG). MAG is essential to maintaining a healthy peripheral nervous system. The disorder is predominantly characterized by distal sensory loss in the extremities (hands and feet), a tingling sensation in the affected limbs, a mild to moderate tremor, and poor balance which can lead to difficulty walking.

"Amyloid neuropathy" is part of "amyloidosis", a heterogeneous group of disorders that may present with a diverse spectrum of clinical manifestations. The disorders are characterized by tissue deposition of insoluble, misassembled fibril proteins that ultimately lead to the disruption of normal tissue structure and function. Up to the present time, 30 proteins have been identified as main amyloid fibril components. Depending on the etiology, amyloid deposits can affect a variety of organ systems and can also affect the peripheral motor, sensory and autonomic nerves.

"Systemic mastocytosis" is part of a spectrum of rare diseases characterized by mast cell accumulation in one or more organs. It is classified into three main groups: cutaneous mastocytosis (CM), with involvement limited to the skin; systemic mastocytosis (SM), with infiltration of the skin and other tissues such as the bone marrow, the gastrointestinal tract or nerves causing neuropathic pain.

"Drug induced peripheral neuropathy" is caused by drugs used in current clinical and that may cause a purely sensory or mixed sensorimotor neuropathy. These include antimicrobials, such as isoniazid, ethambutol, ethionamide, nitrofurantoin, and metronidazole; antineoplastic agents, particularly vinca alkaloids; cardiovascular drugs, such as perhexiline and hydrallazine; hypnotics and psychotropics, notable methaqualone; antirheumatics, such as gold, indomethacin, and chloroquine; anticonvulsants, particularly phenytoin; and other drugs.

By "treatment" is meant, according to the present invention, the inhibition of the development of, more particularly the regression of, preferably the disappearance of the neuropathic pain.

By "prevention" is meant, according to the present invention, to prevent or delay the appearance of the neuropathic pain.

The treatment or prevention according to the invention applies to humans or animals.

By "topical" is meant, according to the present invention, that the compound or composition according to the invention will be administered by application on the skin surface or the mucous membranes.

The topical composition according to the invention may in particular be in any form allowing topical application, such as a cream, a gel, an ointment, a solution, a lotion, a spray, an aerosol spray, an aerosol foam or a patch. Preferably, the composition according to the invention will be in cream or gel form.

In an embodiment, the topical composition of the invention can be applied to the skin by an applicator, such as a roll-on, a stick, an impregnated wipe or an impregnated glove.

In an embodiment, the composition of the invention can also be dispensed from a pump pack or from an aerosol container.

In an embodiment, the topical composition of the invention comprises a compound according to the invention in a concentration from 0.1 to 30%, in particular from 5% to 30%, more particularly from 10% to 20%, by weight relative to the weight of the final composition.

In an embodiment, the topical composition of the invention comprises a compound according to the invention in a concentration of 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19% , 20%, 21%, 22%, 23%, 24%, 25%, 26%, 27%, 28%, 29% or 30% by weight relative to the weight of the final composition.

In a preferred embodiment, the topical composition of the invention comprises a compound according to the invention in a concentration of 10%.

In another preferred embodiment, the topical composition of the invention comprises a compound according to the invention in a concentration of 20%.

In an embodiment, the topical composition of the invention is formulated into unit dose form.

The compound or topical composition according to the invention will be administered one or more times per day, the duration being variable according to the pain intensity and easily adjustable by the person skilled in the art or the practitioner.

In an embodiment, the topical composition of the invention is applied to the affected area once daily, twice daily, three times daily, once every second day, three times weekly, twice weekly or once weekly.

In an embodiment, the topical composition of the invention is applied before a pain episode.

In an embodiment, the topical composition of the invention is applied during a pain episode.

In an embodiment, the topical composition comprises mepyramine in a concentration of 10% for treating or preventing a neuropathic pain caused by a disease chosen from the group comprising erythromelalgia, chemotherapy-induced peripheral neuropathy (CIPN), hereditary neuropathy with pressure palsies (HNPP), algoneurodystrophy, diabetic neuropathy, anti-MAG peripheral neuropathy, amyloid neuropathy, systemic mastocytosis and drug-induced peripheral neuropathy.

In an embodiment, the topical composition comprises mepyramine in a concentration of 20% for treating or preventing a neuropathic pain caused by a disease chosen from the group comprising erythromelalgia, chemotherapy-induced peripheral neuropathy (CIPN), hereditary neuropathy with pressure palsies (HNPP), algoneurodystrophy, diabetic neuropathy, anti-MAG peripheral neuropathy, amyloid neuropathy, systemic mastocytosis and drug-induced peripheral neuropathy.

By "pharmaceutically acceptable excipient" is meant, according to the invention, an excipient that is compatible with the other ingredients of the composition and that produces no adverse effect, allergic reaction or other undesirable reaction when it is administered to a human or an animal.

According to the invention, by "excipient" is meant in particular one or more surfactants, for example macrogols, hydroxy stearates, ethoxylated fatty acid esters, ethoxylated fatty alcohols; one or more solvents, such as for example octyldodecanol, propylene glycol dicaprylocaprate; one or more hydrosoluble polymers, for example PVP, hyaluronic acid or sodium hyaluronate; one or more thickeners such as for example natural or semisynthetic gums; one or more gelling agents, for example carbomers; one or more inorganic fillers, for example zinc oxide, talc, clays; one or more emulsifiers such as cetearyl alcohol; one or more preservatives, for example phenoxyethanol; one or more antibacterials; one or more antiseptics; one or more antioxidants, for example tocopherol acetate; one or more chelating agents, for example EDTA; one or more pigments; one or more fragrances; one or more colorants; one or more pH adjustors such as salts, acids, bases; or a mixture thereof.

In an embodiment, the topical composition of the invention further comprises a penetration enhancer.

By "penetration enhancer" is meant, according to the invention, a chemical substance that promotes the transdermal penetration of the drug applied at the surface of the skin. For example, a penetration enhancer can be selected among alcohols, amides, esters, glycols, fatty acids, pyrrolidones, sulfoxides, surfactants, terpenes, urea, cyclodextrins, water, vitamin E or phospholipids.

In an embodiment, the topical composition further comprises a base allowing transdermal diffusion of said compound. In particular, such a base may be selected from the group comprising Excipial Hydrocrème^{®}, Codexial Obase^{®}, Pentravan^{®}, Pentravan^{®} Plus, Phytobase^{®}, Lipovan^{®} and Pluronic Lecithin Organogel (PLO), preferably Pentravan^{®}, preferably Excipial Hydrocrème^{®} or Codexial Obase^{®}.

In an embodiment, the topical composition of the invention comprises mepyramine in a concentration from 0.1 to 30%, preferably in a concentration of 10%, more preferably in a concentration of 20%, by weight relative to the weight of the final composition.

In an embodiment, the topical composition of the invention comprises mepyramine in a concentration from 0.1 to 30%, preferably in a concentration of 10%, more preferably in a concentration of 20%, by weight relative to the weight of the final composition in a base of Excipial Hydrocrème^{®}.

The following Examples are put forth so as to provide those of ordinary skill in the art with a complete disclosure and description of how to make and use the present invention, and are not intended to limit the scope of what the Inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### FIGURE LEGENDS

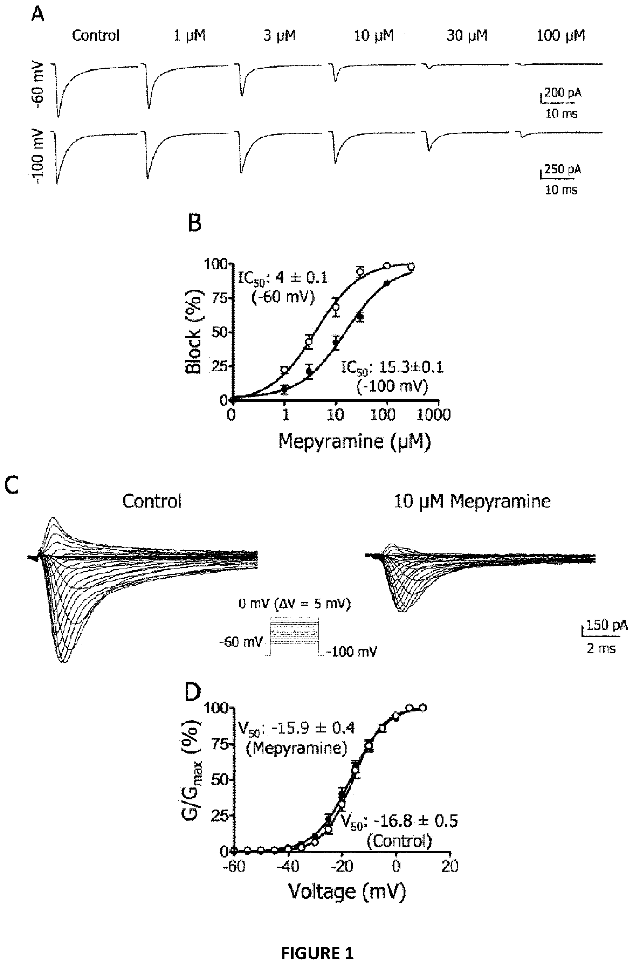

**Figure 1****. A)** Families of human Nav1.7 current traces in the presence of increasing concentrations of mepyramine (0 - 100 µM). The current was elicited by a 50-ms depolarizing pulse to 0 mV from a holding potential of -100 mV (top) or -60 mV (bottom). Voltage steps were applied every 15 s. B) Cumulative dose-response curves for human Nav1.7 inhibition by mepyramine derived from experiments as in A. The IC₅₀ values were 4 ± 0.1 µM at Vh of -60 mV (empty circles) or 15.3 ± 0.1 µM at Vh of -100 mV (filled circles). Each data point is the mean ± SEM (n = 4 - 10). **C**) Representative I-V curve family of hNav1.7 currents recorded using a protocol (inset) where cells were depolarized to a variety of potentials from a holding potential of -100 mV. **D)** Normalized human Nav1.7 activation curves obtained from experiments as in **C.** Curves were fit by using a first-order Boltzmann relations (n = 16).

### EXAMPLES

### Example 1 - Mepyramine inhibits heterologously expressed human Nav1.7 channels.

### Methodology

Human embryonic kidney (HEK) 293T cells were cultured as described in Hao et al. (Neuron, 2013, 77:899-914) and were recorded 48 hours after co-transfection with human Nav1.7 (hNav1.7) and green fluorescent protein clones. Patch clamp recordings were performed using borosilicate electrodes (Harvard Apparatus) having a resistance of 2-3 MΩ when filled with the intracellular solution. Recordings were made at room temperature (20-24°C) using an Axopatch 200B amplifier (Axon Instruments), filtered at 1 kHz, and digitally sampled at 5-20 kHz. Data acquisition was performed with pClamp 10.2, and data were analyzed using Prism 4 software.

For voltage clamp protocols, currents were leak-subtracted by using a P/6 protocol. Voltage errors were minimized using 75-80% series resistance compensation. Intracellular pipette solution consisted of (mM): 130 CsCl, 10 Hepes, 8 NaCl, 0.4 NaGTP, 4 MgATP, 1 MgCl₂, 1 CaCl₂ and 10 EGTA (adjusted to pH7.3 with CsOH, ∼300 mOsm/l). The extracellular solution had a reduced concentration for Na+ (in mM): 55 TEACl, 100 Sucrose, 30 NaCl, 3 KCl, 2.5 CaCl₂, 1 MgCl₂, 10 HEPES, 10 glucose (pH 7.35, 300 mOsm/l). Stock solution of Mepyramine (50 µM, Sigma) was dissolved in distilled water. Cells were perfused with bath solution at flow rate of 5 mL/min.

The dose-response curve for mepyramine was fitted with the Hill equation: I/Imax = [M]^{nH}/(IC₅₀^{nH} + [M]^{nH}), where IC₅₀ is the half-maximal inhibitory concentration and nH the Hill coefficient. The activation curve (G-V) was fitted using Boltzmann function: G/Gmax = 1/(1 + exp^{[(V1/2 - V)/k]}), where G/Gₘₐₓ is the normalized conductance, V_{1/2} is the voltage of half-maximum conductance activation, and k is the steepness factor. Data were presented as mean ± SEM.

Data were tested for normality if n was > 7 and then we used either parametric or nonparametric statistical tests as appropriate. Statistical analysis for sample < 7 used Mann-Whitney non-parametric test and Wilcoxon's test. Behavioral assays were analyzed using Kruskal-Wallis test with Dunns posttest or 2-way analysis of variance test followed by a Bonferroni posttest. Figure legends specify which test was used for specific experiments. Difference was considered significant if *p* < 0.05.

### Results

As shown in Figure 1, Mepyramine inhibits heterologously expressed human Nav1.7 channels.

### Example 2 - A topical treatment with Mepyramine reduces neuropathic pain.

Fifteen patients suffering from neuropathy with no positive result from anti-depressant or anti-epileptic treatments *per os* and no efficiency of topical amitriptyline or topical ambroxol were treated with a cream of Mepyramine 10% at two applications per day.

### Patients :

Patient 1, male, 4 years old, erythromelalgia
Patient 2, female, 10 years old, erythromelalgia
Patient 3, male, 15 years old, erythromelalgia
Patient 4, male, 48 years old, chemotherapy-induced peripheral neuropathy (CIPN)
Patient 5, female, 57 years old, hereditary neuropathy with pressure palsies (HNPP)
Patient 6, female, 19 years old, algoneurodystrophy
Patient 7, female, 38 years old, erythromelalgia
Patient 8, male, 16 years old, erythromelalgia
Patient 9, female, 60 years old, erythromelalgia
Patient 10, male, 58 years old, Chemotherapy-induced peripheral neuropathy (CIPN)
Patient 11, male, 80 years old, amyloid neuropathy
Patient 12, female, 62 years old, diabetic neuropathy
Patient 13, female, 72 years old, anti-MAG neuropathy
Patient 14, female, 52 years old, mastocytosis induced-neuropathy
Patient 15, male, 66 years old, drug induced neuropathy

For all of tested patients, it was observed a dramatic relief of neuropathic pain with burning, tingling, electric shocks disappearance in less than 7 days and a reduction of 4 points on average at 1 month (using VAS pain score between 0 (no pain) and 10 (worst pain imaginable). For the 4 young patients with neuropathic pain who experience peak burns several times per day, the application of topical mepyramine during painful peaks allows the crisis to fade in less than 10 minutes.

## Claims

1. A compound selected from mepyramine, prodrugs thereof and pharmaceutically acceptable salts thereof, for use in the topical treatment or prevention of neuropathic pain.

2. A topical composition comprising a compound selected from mepyramine, prodrugs thereof and pharmaceutically acceptable salts thereof, for use in the treatment or prevention of a neuropathic pain.

3. The topical composition for use according to claim 2, wherein said neuropathic pain is caused by a disease chosen from the group comprising erythromelalgia, chemotherapy-induced peripheral neuropathy (CIPN), hereditary neuropathy with pressure palsies (HNPP), algoneurodystrophy, diabetic neuropathy, anti-MAG peripheral neuropathy, amyloid neuropathy, systemic mastocytosis and drug-induced peripheral neuropathy.

4. The topical composition for use according to any one of claims 2-3, wherein the composition is in the form of a solution, a gel, a cream, an ointment, a lotion, a spray, an aerosol spray, an aerosol foam or a patch.

5. The topical composition for use according to any one of claims 2-4, wherein the compound is in a concentration from 0.1% to 30%, preferably in a concentration of 10%, more preferably in a concentration of 20%, by weight relative to the weight of the final composition.

6. The topical composition for use according to any one of claims 2-5, wherein the topical composition is formulated into unit dose form.

7. The topical composition for use according to any one of claims 2-6, wherein the topical composition is applied to the affected area once daily, twice daily, three times daily, once every second day, three times weekly, twice weekly or once weekly.

8. The topical composition for use according to any one of claims 2-7, further comprising a penetration enhancer.
